# EUROPEAN PATENT SPECIFICATION

(11) **EP 3 626 706 B1**
(45) Date of publication and mention of the grant of the patent: **02.06.2021**
(21) Application number: 18195662.4
(22) Date of filing: 20.09.2018
(51) Int. Cl.: C07C 277/02, C07C 279/02, C07B 63/00, C07C 277/06

(54) **METHOD FOR INCREASING THE GUANIDINE CARBONATE YIELD IN A COMBINED MELAMINE PROCESS AND GUANIDINE CARBONATE TREATMENT PROCESS**
VERFAHREN ZUR ERHÖHUNG DER GUANIDINCARBONATAUSBEUTE IN EINEM KOMBINIERTEN MELAMINVERFAHREN UND GUANIDINCARBONATBEHANDLUNGSVERFAHREN
PROCÉDÉ POUR AUGMENTER LE RENDEMENT DU CARBONATE DE GUANIDINE DANS UN PROCESSUS DE MÉLAMINE COMBINÉ ET PROCÉDÉ DE TRAITEMENT DU CARBONATE DE GUANIDINE

(43) Date of publication of application: 25.03.2020
(73) Proprietor: Borealis Agrolinz Melamine GmbH, 4021 Linz (AT)
(72) Inventor: GABRIEL, Herbert, 4021 Linz (AT); DICKE, René, 4021 Linz (AT)
(74) Representative: Maikowski & Ninnemann Patentanwälte Partnerschaft mbB

(56) References cited:
- US-A- 2 658 891
- US-A- 2 658 891
- US-A- 3 952 057
- US-A- 3 952 057
- US-A- 3 984 471
- US-A- 3 984 471

## Description

The present invention relates to a method for increasing the guanidine carbonate yield in a combined melamine process and guanidine carbonate treatment process and a plant for carrying out this method.

### Description

Guanidine carbonate (GC) is increasing in demand due to a broadening range of applications, especially in the cosmetic industry. Worldwide, there are not many producers of guanidine carbonate and therefore it is strategically seen as a very important product.

There are various ways of guanidine carbonate production. According to DE938843B or US2221478A guanidine salts can be obtained from dicyandiamide and ammonia salts. According to DE1593472 A1 Guanidine carbonate can be synthesised from cyanamide with ammonium carbonate. Here, ammonium carbonate is mixed with aqueous ammonia and heated to a temperature between 130 and 140 °C. Then the cyanamide is added within approx. 6 minutes in a ratio of cyanamide : ammonium carbonate = 1 : 1,9. After 15 minutes the reaction mixture is cooled down to room temperature. If 120 °C is used as reaction temperature then the reaction time must be increased to 40 minutes. The yield of this synthesis is 90 %.

US 3,952,057 and US 3,984,471 describe processes for the preparation of guanidine carbonate, wherein the aqueous solution of urea and its pyrolysis products as well as ammonia and carbon dioxide is heated to 80°C to 130°C at atmospheric pressure, evaporated and the precipitate is separated.

According to US 2,658,891 a reaction mass consisting of calcium cyanamide, ammonia and carbon dioxide is heated at a temperature of at least about 65°C and a pressure of at least 450 psi to produce a reaction mass containing a guanidine salt and melamine.

CN1560031 also deals with the synthesis of GC from cyanamide but uses ammonia and carbon dioxide for the reaction. Here, guanidine carbonate is synthesized by reacting an aq. soln. of cyanamide directly with carbon dioxide and ammonia. The process comprises introducing carbon dioxide and ammonia at the ratio of 0.14 - 20:1 into a reactor, adding the aq. soln. of cyanamide, and reacting to get guanidine carbonate. Reaction conditions and control operation required by the method are very simple. By continuously adding an aqueous solution of cyanamide dropwise into the mixture of carbon dioxide and ammonia, cyanamide can be converted to guanidine carbonate quickly in the reaction system. The method has the advantages of simple process, low cost for raw materials, no toxic org. solvent used, and little environmental pollution

Guanidine carbonate may also be obtained as a by-product of the melamine low-pressure process as a white powder. This is described for example in AT317918B and AT336035B both referring to methods for obtaining guanidine carbonate in the melamine low pressure process.

The object of the present invention is therefore to provide a method which allows for increasing the guanidine carbonate yield in an existing melamine plant that is connected to a guanidine carbonate work-up or treatment process,

This object is solved by a method according to claim 1 and a plant according to claim 15.

Accordingly, a method for increasing the guanidine carbonate yield in a combined melamine process and guanidine carbonate treatment process is provided,
wherein cyanamide and/ or dicyandiamide is/are added to
a) a melamine mother liquor (MML) obtained in the melamine process,
b) a guanidine carbonate mother liquor (GCML) obtained in the guanidine carbonate treatment process, or
c) a mixture of a melamine mother liquor (MML) and a guanidine carbonate mother liquor (GCML).

Guanidine carbonate is formed as by-product in the melamine process. The key invention is the additional formation of guanidine carbonate (direct synthesis of guanidine carbonate) by addition of cyanamide or dicyandiamide in the melamine process.

The present method uses the two cycles of a melamine process and guanidine carbonate treatment process to increase the guanidine carbonate yield by direct introduction of cyanamide or dicyandiamide in the melamine process and / or guanidine carbonate process. A direct addition of cyanamide or dicyandiamide in combination with ammonium carbonate and ammonia (ammonium carbonate and ammonia are already present in the mother liquor, but can be added also additionally) form additional guanidine carbonate. Cyanamide and/ or dicyandiamide can be added to already existing vessels or device within both processes or additional equipment may be installed.

In a preferred embodiment of the present method ammonium carbonate is added to the melamine mother liquor (MML), the guanidine carbonate mother liquor (GCML) or the mixture thereof, in particular to the guanidine carbonate mother liquor (GCML), additionally to cyanamide and/ or dicyandiamide. By adding ammonium carbonate the formation of guanidine carbonate can be improved even further since the reaction equilibrium is influenced in favor of guanidine carbonate.

In general a melamine low pressure process comprises the steps of:
- introducing urea and ammonia in a decomposer;
- introducing decomposed mixture containing isocycanic acid into the fixed-bed catalytic reactor (preferred catalyst Al₂O₃);
- introducing reaction mixture leaving the fixed bed catalytic reactor into a quencher;
- removal of melamine from the quenching mixture leaving the quencher by centrifugation, and
- providing melamine mother liquor (MML).

In the decomposer liquid urea is decomposed in a fluidized bed reactor (370 °C) to ammonia and isocyanic acid (Scheme 1). Ammonia is used as the fluidizing gas.

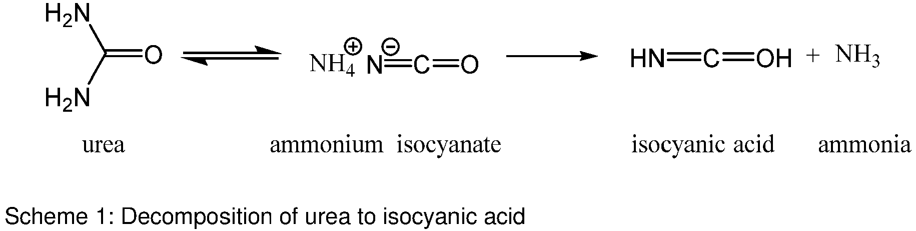

The gas stream is then fed to the fixed-bed catalytic reactor, where isocyanic acid is converted to melamine. Melamine is recovered from the reaction gas by quenching with water and mother liquor from the centrifuges. The overall reaction mechanism is not yet fully understood, but isocyanic acid from the decomposition of urea is believed to be catalytically disproportionated into carbon dioxide and carbodiimide (Scheme 2) followed by the conversion to cyanamide (Scheme 3).

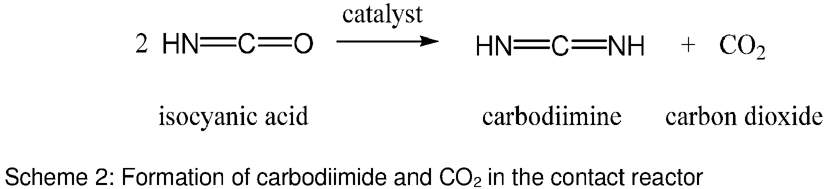

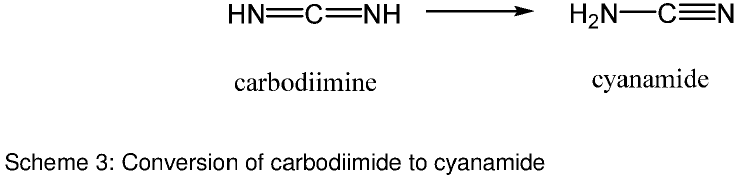

The next step is the trimerisation of cyanamide to melamine (Scheme 4). This step seems to happen immediately after the formation of cyanamide.

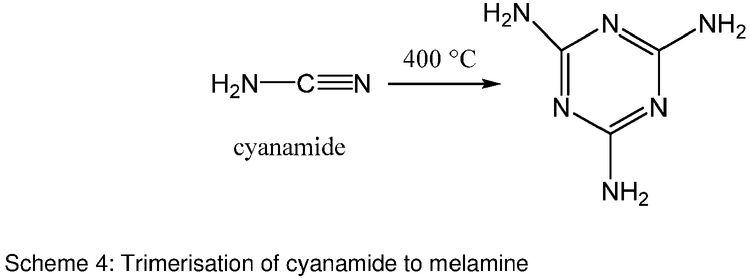

The quencher is specially designed to work quickly, thereby preventing significant hydrolysis of melamine to ammelide and ammeline. In the quencher an aqueous melamine suspension is used to cool the reaction gases to 78 °C were solid melamine is obtained. A continuous circulation is carried out, which results in a 20% melamine suspension. The melamine suspension from the quencher is cooled further to 38 °C to complete the melamine crystallization process. The melamine is subsequently separated (for example by centrifugation) and no further melamine purification is necessary.

Guanidine carbonate is formed as a by-product of the melamine low pressure process. It can be isolated from the melamine mother liquor by heating (to remove ammonia and carbon dioxide, and precipitate the remaining melamine) and filtering. After further concentration, the liquor is cooled and the crystallized guanidine carbonate is separated.

Ammonium carbonate is formed by combining carbon dioxide and aqueous ammonia (Scheme 5). The ammonia is formed during the decomposition of urea and the carbon dioxide is formed in the contact reactor as a by-product of the formation of carbodiimide. In the quenching section water is introduced in the process and then ammonium carbonate is formed.

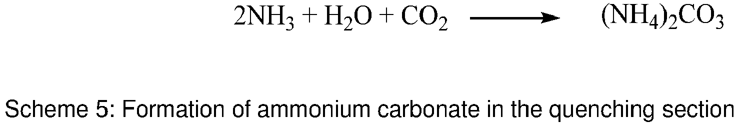

The formed ammonium carbonate can react in the quenching section with cyanamide and guanidine carbonate is formed (Scheme 6).

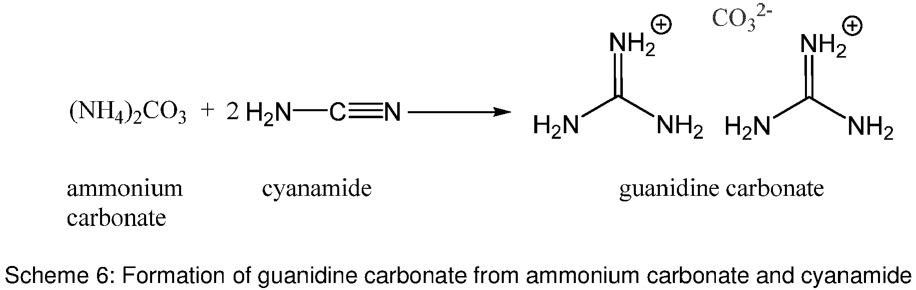

It seems that the formation of guanidine salts by the reaction of cyanamide with ammonium salts is catalyzed by free ammonia. It is assumed that the cyanamide first reacts with ammonia to form guanidinum ion (Scheme 7).

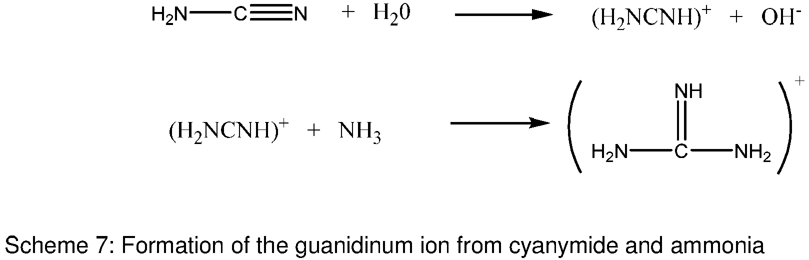

This product is an extremely active base and reacts immediately with the ammonium carbonate to guanidine carbonate (Scheme 8). The temperature should be between 100 and 150 °C.

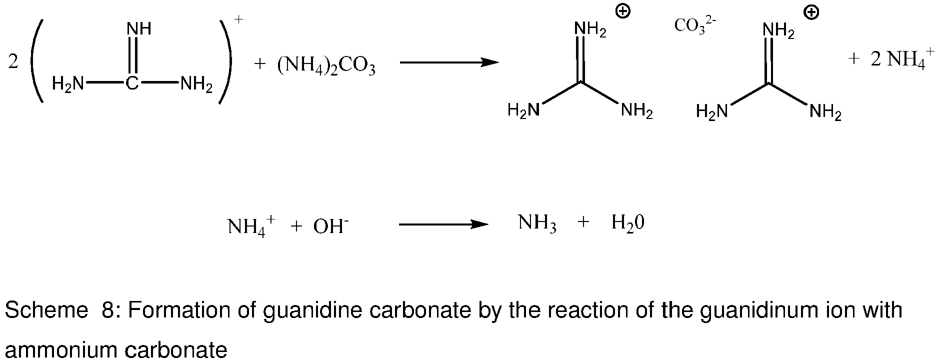

Another possibility how guanidine carbonate can be formed is the direct reaction of aqueous cyanamide with ammonia and carbon dioxide during quenching (Scheme 9).

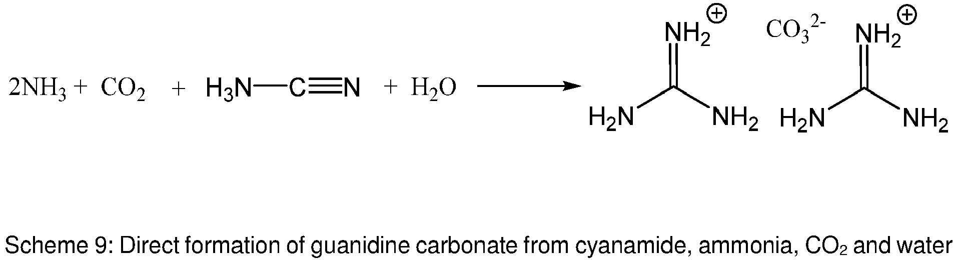

In summary, the melamine mother liquor (MML) containing guanidine carbonate is preferably obtained in a melamine low pressure process comprising the steps:
- introducing urea and ammonia into at least one decomposer for obtaining a decomposed mixture containing isocyanic acid;
- introducing the decomposed mixture containing isocyanic acid into at least one the fixed-bed catalytic reactor wherein a melamine containing reaction mixture is formed;
- introducing the melamine containing reaction mixture into at least one quencher;
- removing melamine suspension (quenching mixture) from the quencher;
- separating melamine from the quenching mixture, preferably by centrifugation), and
- providing the melamine mother liquor (MML).

The melamine mother liquor (MML), i.e. before the addition of cyanamide and/ dicyandiamide and ammonium carbonate, may comprise urea, melamine, ammonia, ammonium carbonate and guanidine carbonate, in particular said liquor may comprise 10 - 150 g/l, preferably 30 -70 g/l urea, 2 - 10 g/l, preferably 4 - 6 g/l melamine, 50 - 300 g/l, preferably 100 - 200 g/l, more preferably 140 -150 g/l ammonia and 5 - 60 g/l, preferably 15-35 g/l guanidine carbonate. For example, the melamine mother liquor (MML) may contain 20 g/l guanidine carbonate, 50 g/l urea, 5 g/l melamine and 140 g/l ammonia.

According to the invention additional cyanamide and/ dicyandiamide and ammonium carbonate or ammonia / carbon dioxide may be added at this point to the melamine mother liquor.

The guanidine carbonate formed in the course of the melamine process is dissolved in the melamine mother liquor. The mother liquor from the melamine filtration is partially used in the quenching process and the rest is worked up to obtain guanidine carbonate in the guanidine carbonate treatment process.

In the subsequent guanidine carbonate treatment process the melamine mother liquor is heated to remove the rest of ammonia and carbon dioxide and then concentrated, for example up to 20 % volume. Most of the dissolved melamine precipitates by cooling and is filtered off. Subsequently, gaseous ammonia is introduced and guanidine carbonate precipitates and can be filtered off.

The guanidine carbonate treatment process may comprise the steps of
- evaporating the melamine mother liquor (MML) from the melamine process in an evaporation step, wherein melamine and other impurities precipitate;
- removing precipitated melamine (and other impurities), preferably by filtration;
- adding ammonia to the mother liquor for precipitating guanidine carbonate and removing precipitated guanidine carbonate, preferably by centrifugation, and
- providing the guanidine carbonate mother liquor (GCML), which is reintroduced into the evaporation step.

The guanidine carbonate mother liquor (GCML), i.e. before the addition of cyanamide and/ dicyandiamide and optionally ammonium carbonate to the guanidine carbonate mother liquor (GCML), comprises urea, melamine, ammonia and guanidine carbonate, preferably said liquor comprises 30 - 150 g/l, preferably 70-100 g/l urea, 2 - 8 g/l, preferably 3-5 g/l melamine, 100 - 350 g/l, preferably 180 - 270 g/l, more preferably 200-250 g/l ammonia and 10 - 60 g/l, preferably 25-35 g/l guanidine carbonate, for example 32 g/l guanidine carbonate.

Fresh melamine mother liquor may be added to the guanidine carbonate mother liquor before or after guanidine carbonate mother liquor is mixed with cyanamide and/ dicyandiamide and ammonium carbonate. In one variant the fresh melamine mother liquor may be added before or during the evaporation step of melamine mother liquor and guanidine carbonate mother liquor.

In an embodiment of the present method - after the addition of cyanamide and/ dicyandiamide and ammonium carbonate which can be also done either by adding ammonium carbonate or carbon dioxide and ammonia to the guanidine carbonate mother liquor (GCML) - the guanidine carbonate mother liquor (GCML) containing cyanamide and/ dicyandiamide and optionally ammonium carbonate is mixed with fresh melamine mother liquor (MML) and the mixture is reintroduced into the evaporation step.

In yet another embodiment of the present method the guanidine carbonate mother liquor (GCML) is mixed with fresh melamine mother liquor (MML), subsequently cyanamide and/ dicyandiamide and optionally ammonium carbonate are added and the mixture is reintroduced into the evaporation step.

In still another embodiment of the present method cyanamide and/ dicyandiamide and optionally ammonium carbonate are added to melamine mother liquor (MML), subsequently guanidine carbonate mother liquor (GCML) is added and the mixture is reintroduced into the evaporation step.

In case no additional ammonium carbonate is added the melamine mother liquor and the guanidine carbonate mother liquor are mixed before the addition of cyanamide and/ dicyandiamide.

It is also preferred, if the evaporation step of the melamine mother liquor (MML) from the melamine process (as part of the guanidine carbonate treatment process) is carried out at 80°C-150°C, preferably between 90°C - 125°C, more preferably 100°C-120°C, at a pressure between 0.4-0.7 bar for a time of 1-6 h, preferably 2-4 h. The evaporation time depends on the evaporation temperature. For example, with an evaporation temperature of 130°C an evaporation time if 2-4h is sufficient. In case of an evaporation temperature of about 110°C the evaporation time may be between 5-6 h.

After evaporation and removal of any precipitated melamine the remaining liquor is cooled down, preferably to 45°C or lower, even as low as room temperature.

In still another embodiment of the present method, in particular of the guanidine carbonate treatment cycle, 15-30 wt%, preferably 20-25wt% gaseous or liquid ammonia are added to the mother liquor for precipitation of guanidine carbonate, wherein the addition of gaseous ammonia is preferred. The addition of ammonia increases the pH-value thereby reducing solubility of guanidine carbonate.

In a further embodiment of the present method 2.5- 50 g/l cyanamide and/or 2.5-50 g/l dicyandiamide, preferably 5-25 g/l, more preferably 10-20 g /l cyanamide and/or 5-25 g/l, more preferably 10-20 g/ l dicyandiamide are added.

In one embodiment cyanamide may be added in an amount between 0.05-1.2 mol/l, preferably 0.1-0.6 mol/l, more preferably between 0.2-0.5 mol/l.

In another embodiment dicyandiamide may be added in an amount between 0.03-0.6 mol/l, preferably between 0.05-0.3mol/l, preferably between 0.06-0.15 mol/l, for example dicyandiamide may be added in an amount between 0.1-0.3 mol/l.

The amount of ammonium carbonate that may be added to the respective mother liquors can be between 2.5-100 g/l, preferably 5-50 g/l, more preferably 10-30 g/l.

In an even more preferred embodiment of the present method 2.5-25 g/l cyanamide and/or 2.5-25 g/l dicyandiamide and 2.5-50 g/l ammonium carbonate are added to the melamine mother liquor (MML).

In another preferred embodiment of the present method 5-50 g/l cyanamide and/or 5-50 g/l dicyandiamide and 5-100 g/l ammonium carbonate are added to the guanidine carbonate mother liquor (GCML).

In a further variant of the present method the addition of cyanamide and/ dicyandiamide and optionally ammonium carbonate to the melamine mother liquor (MML), a guanidine carbonate mother liquor (GCML) or a mixture thereof is carried out at a temperature between 100°C and 150°C, preferably between 110°C and 140°C, for 15 to 120 min. Said temperature range is also applied in all subsequent reaction steps, in particular in the guanidine carbonate treatment process.

The above described method may be carried out in existing melamine and guanidine carbonate treatment plants. In one embodiment cyanamide, dicyandiamide and optionally ammonium carbonate may be introduced into a tank containing melamine mother liquor (MML), into a tank containing a guanidine carbonate mother liquor (GCML), and/or into an evaporation reactor containing a mixture of both melamine mother liquor (MML) and a guanidine carbonate mother liquor (GCML). The addition of cyanamide, dicyandiamide and optionally ammonium carbonate to MML tank and/or GCML tank are preferred.

However, it is also conceivable and possible to introduce cyanamide, dicyandiamide and optionally ammonium carbonate (and also fresh melamine mother liquor) into a separate reactor, such a tubular reactor, which is located between the tank containing a guanidine carbonate mother liquor (GCML) and the evaporation tank, i.e. downstream of the GCML tank and the evaporation reactor.

In such a case, a plant is provided that comprises
- devices (or installations) for carrying out the melamine process, and
- devices (or installations) for carrying out the guanidine carbonate process with at least one evaporation reactor for evaporating the guanidine carbonate mother liquor (GCML) and melamine mother liquor (MML),
- wherein upstream of the at least one evaporation reactor an additional reactor, in particular a tubular reactor or a stirred reactor (batch or continuous) is provided, and
- wherein melamine mother liquor (MML), guanidine carbonate mother liquor (GCML) or a mixture thereof is fed into the at least one additional reactor.

In an embodiment the plant comprises
- devices (or installations) for carrying out the melamine process with at least one melamine mother liquor tank for melamine mother liquor (MML) obtained after separating melamine from a melamine suspension, for example by centrifugation, and
- devices (or installations) for carrying out the guanidine carbonate process with at least one guanidine carbonate mother liquor tank for guanidine carbonate mother liquor (GCML) obtained after separating guanidine carbonate from a guanidine carbonate suspension, for example by centrifugation; and with at least one evaporation reactor located downstream of the guanidine carbonate mother liquor tank;
- wherein between the least one guanidine carbonate mother liquor tank and the at least one evaporation reactor an additional reactor, in particular a tubular reactor or a stirred reactor (batch or continuous) is provided, and
- wherein the melamine mother liquor tank is also in connection with said additional reactor for feeding the melamine mother liquor from the melamine mother liquor tank into said additional tank in the guanidine carbonate process.

As mentioned, the additional reactor may be a tubular reactor, in particular a continuous tubular reactor or a longer tube. The residence time of the liquor in the reactor may be between 15 and 360 min. Any other reactor type, such as a batch reactor or continuous stirred reactor, may also be applicable as long as said residence time can be maintained.

In summary, there are at least four different locations within the plant system, at which cyanamide, dicyandiamide and optionally ammonium carbonate may be added to the melamine mother liquor (MML), guanidine carbonate mother liquor or a mixture of both. In a first embodiment cyanamide, dicyandiamide and optionally ammonium carbonate are introduced into the melamine mother liquor tank. In a second embodiment cyanamide, dicyandiamide and optionally ammonium carbonate are introduced into the guanidine carbonate mother liquor tank. In a third embodiment cyanamide, dicyandiamide and optionally ammonium carbonate are introduced into the evaporation reactor. And in a fourth embodiment cyanamide, dicyandiamide and optionally ammonium carbonate are introduced into the tubular line (or tubular reactor) or batch reactor upstream or upfront of the guanidine carbonate mother liquor evaporation reactor.

As stated previously, guanidine carbonate mother liquor may be mixed with fresh melamine mother liquor before or after adding cyanamide, dicyandiamide and optionally ammonium carbonate. Thus, in one embodiment the fresh melamine mother liquor may be introduced into the evaporation reactor. In a second embodiment he fresh melamine mother liquor may be introduced into the tubular line (or tubular reactor) or batch reactor between guanidine carbonate mother liquor tank and mother liquor evaporation reactor.

The invention is explained in more detail by means of the following examples with reference to the Figures. It shows:

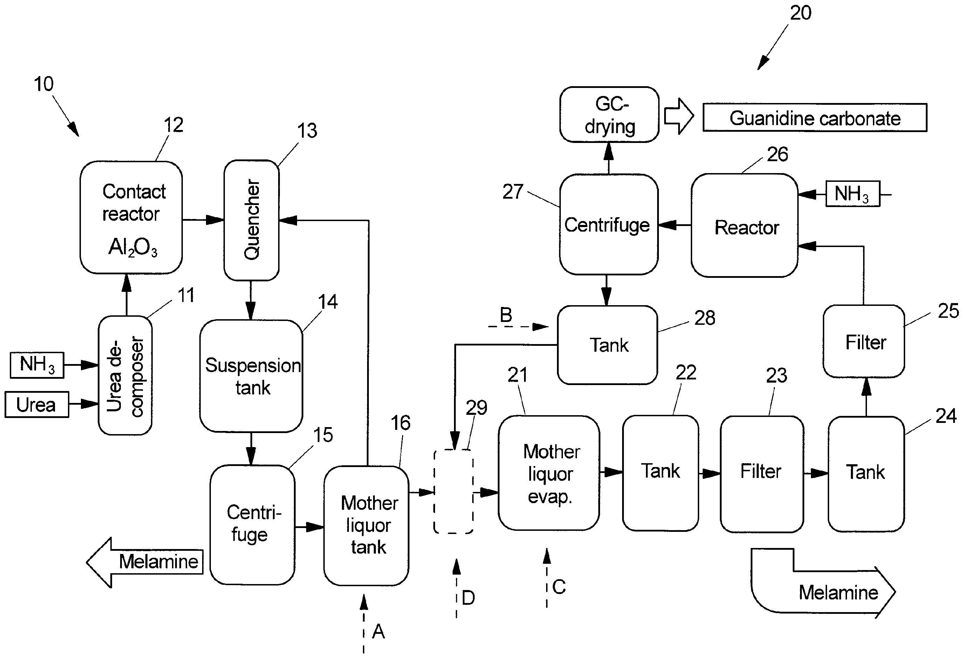

- Figure 1: scheme of a combined melamine low pressure process and a guanidine carbonate treatment process.

Figure 1 shows a melamine low pressure process 10 that is connected to a process for isolating guanidine carbonate 20.

The central steps of a melamine low pressure process are: introducing urea and ammonia in a decomposer 11; introducing decomposed mixture containing isocycanic acid into the fixed-bed catalytic reactor 12; introducing reaction mixture leaving the fixed bed catalytic reactor 12 into a quencher 13; removal of melamine from the quenching mixture leaving the quencher 13 by centrifugation 15; and providing melamine mother liquor (MML) in a mother liquor tank 16.

Urea and ammonia are introduced in the decomposer 11 and decomposed in a fluidised bed reactor (370 °C) to ammonia and isocyanic acid. Ammonia is used as the fluidizing gas. Heat required for the decomposition is supplied to the reactor by hot molten salt circulated through internal heating coils.

The gas stream leaving the decomposer 11 is then fed to the fixed-bed catalytic reactor 13, where isocyanic acid is converted to melamine at ca. 450 °C and near-atmospheric pressure in the presence of Al₂O₃ as catalyst.

Melamine is recovered from the reaction gas by quenching with water and mother liquor in quencher 13. The quencher 13 is designed to work quickly, thereby preventing significant hydrolysis of melamine to ammelide and ammeline. In the quencher 13 an aqueous melamine suspension is used to cool the reaction gases to 78 °C whereby solid melamine is obtained. A continuous circulation is carried out, which results in a 20% melamine suspension.

The melamine suspension from the quencher 13 is cooled further to 38 °C in the suspension tank 14 to complete the melamine crystallization process. Subsequently, melamine is separated from the suspension in centrifuge 15.

Any guanidine carbonate formed in the course of the melamine process 10 (as described previously above) is dissolved in the melamine mother liquor. The mother liquor from the melamine filtration is partially used in the quenching process and the rest is worked up to obtain guanidine carbonate in the guanidine carbonate treatment process 20.

Here, the melamine mother liquor (MML) is transferred from the MML tank 16 to the evaporation tank 21 where the MML mixes with the recycled guanidine carbonate mother liquor (GCML). The combined mother liquors (MML and GCML) are heated in the evaporation reactor 21 to remove the rest of ammonia and carbon dioxide and simultaneously concentrated, for example up to 20 % volume.

The concentrated combined MML+GCML are transferred to a further tank 22 for cooling off whereby most of the melamine contained therein precipitates. The precipitated melamine is filtered off using filter 23. After filtration the mother liquor goes to another tank 24 and is filtered again with filter 25. In reactor 26, gaseous ammonia is introduced whereby guanidine carbonate precipitates which can be separated in a centrifuge 27.

Ammonia is added to precipitate the guanidine carbonate caused by an increase of the pH-value. The higher the ammonia content the better the precipitation of guanidine carbonate. The addition of ammonia leads to a temperature increase of the solution due to the high heat caused by ammonia absorption. Therefore another cooling step is necessary to cool the solution down to 28°C and However, the precipitation temperature should be above the temperature of melamine precipitation (currently 25 °C) otherwise not precipitated melamine or other impurities would be precipitated with the guanidine carbonate.

The guanidine carbonate mother liquor leaving the centrifuge 27 is transferred to another tank 28, may pass an additional reactor or tube 29 and is subsequently reintroduced into the mother liquor evaporation reactor 21.

Cyanamide, dicyandiamide and optionally ammonium carbonate may be added at different locations (depicted as dashed arrows A-D in Figure 1).

In a first setting A cyanamide, dicyandiamide and optionally ammonium carbonate are introduced into the melamine mother liquor tank 16.

In a second setting B cyanamide, dicyandiamide and optionally ammonium carbonate are introduced into the guanidine carbonate mother liquor tank 28.

In a third setting C cyanamide, dicyandiamide and optionally ammonium carbonate are introduced into the mother liquor evaporation reactor 21.

And In a fourth setting D cyanamide, dicyandiamide and optionally ammonium carbonate are introduced into the tubular line, tubular or batch reactor 29 installed between tanks 28 and 21.

### Examples

The following experiments were carried out in an EasyMax autoclave. Melamine mother liquor samples were taken from the melamine mother liquor tank 16 Guanidine carbonate mother liquor samples were taken from Guanidine carbonate mother liquor tank 28

The melamine mother liquor from tank 16 contains 50 g/l urea, 5 g/l melamine, 140 g/l ammonia and 20 g/l guanidine carbonate.

The guanidine carbonate mother liquor from tank 28 contains 90 g/l urea, 4 g/l melamine, 240 g/l ammonia and 32 g/l guanidine carbonate.

Cyanamide or dicyandiamide and ammonium carbonate were added in different concentrations to the mother liquors and heated to 120 °C for 60 min with a stirring rate of 150 rpm. The samples were cooled to room temperature and the Guanidine Carbonate (GC) content was analysed by HPLC. The results are summarized in the following tables.

Table 1 shows the addition of cyanamide and ammonium carbonate to mother liquor from tank 16

**Table 1**

| | **Cyanamide [g]** | **Ammon.carb [g]** | **Rea. time [min]** | **Temp. [°C]** | **Yield [g/l]** | **Conversion rate [%]** |
|---|---|---|---|---|---|---|
| 0 | --- | --- | --- | --- | 22,3 | --- |
| 1 | 2.5 | 5 | 60 | 120 | 52,6 | 36 |
| 2 | 5 | 5 | 60 | 120 | 63,2 | 24.3 |
| 3 | 2.5 | 5 | 60 | 130 | 51,5 | 34.8 |
| 4 | 2.5 | 5 | 120 | 120 | 49,7 | 32.5 |

Table 2 shows the addition of dicyandiamide and ammonium carbonate to mother liquor from tank 16.

**Table 2**

| | **Dicyandiamide [g]** | **Ammon.carb [g]** | **Rea. time [min]** | **Temp. [°C]** | **Yield [g/l]** | **Conversion rate [%]** |
|---|---|---|---|---|---|---|
| 0 | --- | --- | --- | --- | 22,3 | --- |
| 1 | 2.5 | 5 | 60 | 120 | 28,3 | 7.1 |
| 2 | 5 | 5 | 60 | 120 | 39,8 | 10,4 |
| 3 | 2.5 | 5 | 60 | 130 | 33,7 | 13.5 |
| 4 | 2.5 | 5 | 120 | 120 | 40,4 | 21.5 |

Table 3 shows the addition of cyanamide and ammonium carbonate to mother liquor from tank 28

**Table 3**

| | **Cyanamide [g]** | **Ammon.carb [g]** | **Rea. time [min]** | **Temp. [°C]** | **Yield [g/l]** | **Conversion rate [%]** |
|---|---|---|---|---|---|---|
| 0 | --- | --- | --- | --- | 22,9 | --- |
| 1 | 1 | 5 | 60 | 120 | 41,0 | 55 |
| 2 | 2 | 5 | 60 | 120 | 47,3 | 37 |
| 3 | 1 | 5 | 60 | 130 | 39,9 | 52 |
| 4 | 1 | 5 | 120 | 120 | 42,1 | 58 |

Table 4 shows the addition of dicyandiamide and ammonium carbonate to mother liquor from tank 28.

| | **Dicyandiamide [g]** | **Ammon.carb [g]** | **Rea. time [min]** | **Temp. [°C]** | **Yield [g/l]** | **Conversion rate [%]** |
|---|---|---|---|---|---|---|
| 0 | --- | --- | --- | --- | 22,9 | --- |
| 1 | 1 | 5 | 60 | 120 | 32,9 | 30 |
| 2 | 2 | 5 | 60 | 120 | 34,3 | 17 |
| 3 | 1 | 5 | 60 | 130 | 35,7 | 39 |
| 4 | 1 | 5 | 120 | 120 | 37,1 | 43 |

The results of the experiments show that with both scenarios, the addition of cyanamide or dicyandiamide, the yield could be increased. Experiments show, the reaction based on cyanamide provides a greater increase in the yield. Nevertheless, the addition of dicyandiamide resulted also in a significant increase in yield.

The ammonia concentration in tank 28 is higher compared to tank 16, which seems to have an influence on the guanidine carbonate formation because of the higher conversion rate at experiments with mother liquor from tank 28. For the experiments with mother liquor from tank 28 also less cyanamide or dicyandiamide was used. The overall yield was smaller compared to the experiments with mother liquor from tank 16, but the conversion rate was nearly twice as high. This can be caused by a dimerization of cyanamide to dicyandiamide, which obviously reacts slower.

## Claims

1. Method for increasing the guanidine carbonate yield in a combined melamine process (10) and guanidine carbonate treatment process (20) by
adding cyanamide and/ or dicyandiamide to
a) a melamine mother liquor (MML) obtained in the melamine process (10), and/or
b) a guanidine carbonate mother liquor (GCML) obtained in the guanidine carbonate treatment process (20), and /or
c) a mixture of a melamine mother liquor (MML) and a guanidine carbonate mother liquor (GCML).

2. Method according to claim 1, **characterized in that** in addition to cyanamide and/ or dicyandiamide ammonium carbonate or alternatively ammonia / carbon dioxide is added to the melamine mother liquor (MML), the guanidine carbonate mother liquor (GCML) or the mixture thereof, in particular to the guanidine carbonate mother liquor (GCML).

3. Method according to claim 1 or 2, **characterized in that** 2.5-50 g /l cyanamide and/or 2.5-50 g/ l dicyandiamide and 2.5-100 g/l ammonium carbonate are added.

4. Method according to one of the preceding claims, **characterized in that** 2.5-25 g/l cyanamide and/or 2.5-25 g/l dicyandiamide and 2.5-50 g/l ammonium carbonate are added to the melamine mother liquor (MML).

5. Method according to one of the claims 1 - 3, **characterized in that** 5-50 g/l cyanamide and/or 5-50 g/l dicyandiamide and 5-100g/l ammonium carbonate are added to the guanidine carbonate mother liquor (GCML).

6. Method according to one of the preceding claims, **characterized in that** the addition of cyanamide and/ dicyandiamide and optionally ammonium carbonate is carried out at a temperature between 100 and 150°C, preferably between 110 and 140°C, for 15 to 360 min, preferably between 30 and 120 min.

7. Method according to one of the preceding claims, **characterized in that** the melamine mother liquor (MML) is obtained in a melamine low pressure process comprising the steps:
- introducing urea and ammonia into at least one decomposer for obtaining a decomposed mixture containing isocyanic acid;
- introducing the decomposed mixture containing isocycanic acid into at least one the fixed-bed catalytic reactor wherein a melamine containing reaction mixture is formed;
- introducing the melamine containing reaction mixture into at least one quencher;
- removing melamine suspension from the quencher, and
- providing the melamine mother liquor (MML).

8. Method according to one of the preceding claims, **characterized in that** the melamine mother liquor (MML) comprises urea, melamine, ammonia and guanidine carbonate, in particular said liquor comprises 10 - 150 g/l, preferably 30-70 g/l urea, 2 - 10 g/l, preferably 4 - 6 g/l melamine, 50 - 300 g/l, preferably100 - 200 g/l, more preferably 140 - 150 g/l ammonia and 5 - 60 g/l, preferably 15 - 35 g/l guanidine carbonate.

9. Method according to one of the preceding claims, **characterized in that** guanidine carbonate mother liquor (GCML) is obtained in the guanidine carbonate treatment process comprising the steps:
- in an evaporation step evaporating the melamine mother liquor (MML) from the melamine process and the guanidine carbonate mother liquor (GCML) from the guanidine carbonate treatment process, wherein melamine precipitates;
- removing precipitated melamine, preferably by filtration;
- adding ammonia to the mother liquor for precipitating guanidine carbonate and removing precipitated guanidine carbonate, preferably by centrifugation, and
- providing the guanidine carbonate mother liquor (GCML), which is reintroduced into the evaporation step.

10. Method according to claim 9, **characterized in that** after adding cyanamide and/ dicyandiamide and optionally ammonium carbonate to the guanidine carbonate mother liquor (GCML) the guanidine carbonate mother liquor (GCML) containing cyanamide and/ dicyandiamide and optionally ammonium carbonate is mixed with fresh melamine mother liquor (MML) and the mixture is reintroduced into the evaporation step.

11. Method according to claim 9, **characterized in that** the guanidine carbonate mother liquor (GCML) is mixed with fresh melamine mother liquor (MML), subsequently cyanamide and/ dicyandiamide and optionally ammonium carbonate are added and the mixture is reintroduced into the evaporation step.

12. Method according to claim 9, **characterized in that** cyanamide and/ dicyandiamide and optionally ammonium carbonate are added to the melamine mother liquor (MML), subsequently guanidine carbonate mother liquor (GCML) is added and the mixture is reintroduced into the evaporation step.

13. Method according to one of claims 9-12, **characterized in that** 15-30 wt%, preferably 20-25wt% gaseous or liquid ammonia are added to the mother liquor for precipitation of guanidine carbonate.

14. Method according to one of the preceding claims, **characterized in that** the guanidine carbonate mother liquor (GCML) comprises urea, melamine, ammonia and guanidine carbonate, preferably said liquor comprises 300 - 150 g/l, preferably 70-100 g/l urea, 2 - 8 g/l, preferably 2-5 g/l melamine, 100 - 350 g/l, preferably 180 - 270 g/l, more preferably 200-250 g/l ammonia and 10 - 60 g/l, preferably 25-35 g/l guanidine carbonate.

15. Plant for carrying out the method according to one of the preceding claims comprising
- devices for carrying out the melamine process (10) with at least one melamine mother liquor tank (16) for melamine mother liquor (MML) obtained after separating melamine from a melamine suspension, and
- devices for carrying out the guanidine carbonate process (20) with at least one guanidine carbonate mother liquor tank (28) for guanidine carbonate mother liquor (GCML) obtained after separating guanidine carbonate from a guanidine carbonate suspension; and with at least one evaporation reactor (21) located downstream of the guanidine carbonate mother liquor tank (21);
- wherein between the least one guanidine carbonate mother liquor tank (28) and the at least one evaporation reactor (21) an additional reactor (29), in particular a tubular reactor or a stirred reactor is provided, and
- wherein the melamine mother liquor tank (16) is also in connection with said additional reactor (29) for feeding the melamine mother liquor from the melamine mother liquor tank (16) into said additional tank (29) in the guanidine carbonate process.

## Patentansprüche

1. Verfahren zur Erhöhung der Guanidincarbonat-Ausbeute in einem kombinierten Melaminverfahren (10) und Guanidincarbonat-Behandlungsverfahren (20) durch
Zugabe von Cyanamid und/oder Dicyandiamid zu
a) eine Melamin-Mutterlauge (MML), die in dem Melaminverfahren (10) erhalten wird, und/oder
b) eine Guanidincarbonat-Mutterlauge (GCML), die in dem Guanidincarbonat-Behandlungsverfahren (20) erhalten wird, und /oder
c) eine Mischung aus einer Melamin-Mutterlauge (MML) und einer Guanidin-Carbonat-Mutterlauge (GCML).

2. Verfahren nach Anspruch 1, **dadurch gekennzeichnet, dass** der Melamin-Mutterlauge (MML), der Guanidin-Carbonat-Mutterlauge (GCML) oder deren Gemisch, insbesondere der Guanidin-Carbonat-Mutterlauge (GCML), zusätzlich zu Cyanamid und/oder Dicyandiamid Ammoniumcarbonat oder alternativ Ammoniak / Kohlendioxid zugesetzt wird.

3. Verfahren nach Anspruch 1 oder 2, **dadurch gekennzeichnet, dass** 2,5-50 g /l Cyanamid und/oder 2,5-50 g/ l Dicyandiamid und 2,5-100 g/l Ammoniumcarbonat zugesetzt werden.

4. Verfahren nach einem der vorhergehenden Ansprüche, **dadurch gekennzeichnet, dass** der Melamin-Mutterlauge (MML) 2,5-25 g/l Cyanamid und/oder 2,5-25 g/l Dicyandiamid und 2,5-50 g/l Ammoniumcarbonat zugesetzt werden.

5. Verfahren nach einem der Ansprüche 1 - 3, **dadurch gekennzeichnet, dass** der Guanidincarbonat-Mutterlauge (GCML) 5-50 g/l Cyanamid und/oder 5-50 g/l Dicyandiamid und 5-100 g/l Ammoniumcarbonat zugesetzt werden.

6. Verfahren nach einem der vorhergehenden Ansprüche, **dadurch gekennzeichnet, dass** die Zugabe von Cyanamid und/oder Dicyandiamid und gegebenenfalls Ammoniumcarbonat bei einer Temperatur zwischen 100 und 150°C, vorzugsweise zwischen 110 und 140°C, für 15 bis 360 min, vorzugsweise zwischen 30 und 120 min, durchgeführt wird.

7. Verfahren nach einem der vorhergehenden Ansprüche, **dadurch gekennzeichnet, dass** die Melamin-Mutterlauge (MML) in einem Melamin-Niederdruckverfahren erhalten wird, das die Schritte umfasst:
- Einführen von Harnstoff und Ammoniak in mindestens einen Zersetzer, um eine zersetzte Mischung zu erhalten, die Isocyansäure enthält;
- Einführen der zersetzten Mischung, die Isocyansäure enthält, in mindestens einen der katalytischen Festbettreaktoren, wobei eine melaminhaltige Reaktionsmischung gebildet wird;
- Einbringen des melaminhaltigen Reaktionsgemisches in mindestens einen Quencher;
- Entfernen der Melaminsuspension aus dem Quencher, und
- Bereitstellung der Melamin-Mutterlauge (MML).

8. Verfahren nach einem der vorhergehenden Ansprüche, **dadurch gekennzeichnet, dass** die Melamin-Mutterlauge (MML) Harnstoff, Melamin, Ammoniak und Guanidincarbonat enthält, insbesondere 10 - 150 g/l, vorzugsweise 30-70 g/l Harnstoff, 2 - 10 g/l, vorzugsweise 4 - 6 g/l Melamin, 50 - 300 g/l, vorzugsweise 100 - 200 g/l, besonders bevorzugt 140 - 150 g/l Ammoniak und 5 - 60 g/l, vorzugsweise 15 - 35 g/l Guanidincarbonat.

9. Verfahren nach einem der vorhergehenden Ansprüche, **dadurch gekennzeichnet, dass** Guanidincarbonat-Mutterlauge (GCML) in dem Guanidincarbonat-Behandlungsverfahren erhalten wird, das die Schritte umfasst:
- in einem Verdampfungsschritt Verdampfen der Melamin-Mutterlauge (MML) aus dem Melaminverfahren und der Guanidincarbonat-Mutterlauge (GCML) aus dem Guanidincarbonat-Behandlungsverfahren, wobei Melamin ausfällt;
- Entfernen des ausgefällten Melamins, vorzugsweise durch Filtration;
- Zugabe von Ammoniak zur Mutterlauge zur Ausfällung von Guanidincarbonat und Entfernen des ausgefällten Guanidincarbonats, vorzugsweise durch Zentrifugieren, und
- Bereitstellen der Guanidincarbonat-Mutterlauge (GCML), die wieder in den Verdampfungsschritt eingebracht wird.

10. Verfahren nach Anspruch 9, **dadurch gekennzeichnet, dass** nach Zugabe von Cyanamid und/oder Dicyandiamid und gegebenenfalls Ammoniumcarbonat zur Guanidincarbonat-Mutterlauge (GCML) die Cyanamid und/oder Dicyandiamid und gegebenenfalls Ammoniumcarbonat enthaltende Guanidincarbonat-Mutterlauge (GCML) mit frischer Melamin-Mutterlauge (MML) gemischt und das Gemisch erneut in den Verdampfungsschritt eingebracht wird.

11. Verfahren nach Anspruch 9, **dadurch gekennzeichnet, dass** die Guanidincarbonat-Mutterlauge (GCML) mit frischer Melamin-Mutterlauge (MML) gemischt wird, anschließend Cyanamid und/oder Dicyandiamid und gegebenenfalls Ammoniumcarbonat zugegeben werden und die Mischung wieder in den Verdampfungsschritt eingebracht wird.

12. Verfahren nach Anspruch 9, **dadurch gekennzeichnet, dass** der Melamin-Mutterlauge (MML) Cyanamid und/oder Dicyandiamid und gegebenenfalls Ammoniumcarbonat zugesetzt werden, anschließend Guanidincarbonat-Mutterlauge (GCML) zugesetzt wird und das Gemisch wieder in den Verdampfungsschritt eingebracht wird.

13. Verfahren nach einem der Ansprüche 9-12, **dadurch gekennzeichnet, dass** der Mutterlauge zur Ausfällung von Guanidincarbonat 15-30 Gew.-%, vorzugsweise 20-25 Gew.-% gasförmiges oder flüssiges Ammoniak zugesetzt werden.

14. Verfahren nach einem der vorhergehenden Ansprüche, **dadurch gekennzeichnet, daß** die Guanidincarbonat-Mutterlauge (GCML) Harnstoff, Melamin, Ammoniak und Guanidincarbonat umfaßt, wobei die Lauge vorzugsweise 300 - 150 g/l, vorzugsweise 70-100 g/l Harnstoff, 2 - 8 g/l, vorzugsweise 2-5 g/l Melamin, 100 - 350 g/l, vorzugsweise 180 - 270 g/l, noch bevorzugter 200-250 g/l Ammoniak und 10 - 60 g/l, vorzugsweise 25-35 g/l Guanidincarbonat umfaßt.

15. Anlage zur Durchführung des Verfahrens nach einem der vorhergehenden Ansprüche, umfassend
- Vorrichtungen zur Durchführung des Melaminprozesses (10) mit mindestens einem Melamin-Mutterlaugentank (16) für Melamin-Mutterlauge (MML), die nach Abtrennung von Melamin aus einer Melaminsuspension gewonnen wird, und
- Vorrichtungen zur Durchführung des Guanidincarbonat-Verfahrens (20) mit mindestens einem Guanidincarbonat-Mutterlaugentank (28) für Guanidincarbonat-Mutterlauge (GCML), die nach Abtrennung von Guanidincarbonat aus einer Guanidincarbonat-Suspension erhalten wird; und mit mindestens einem Verdampfungsreaktor (21), der dem Guanidincarbonat-Mutterlaugentank (21) nachgeschaltet ist;
- wobei zwischen dem mindestens einen Guanidincarbonat-Mutterlaugentank (28) und dem mindestens einen Verdampfungsreaktor (21) ein Zusatzreaktor (29), insbesondere ein Rohrreaktor oder ein Rührreaktor, vorgesehen ist, und
- wobei der Melamin-Mutterlaugentank (16) auch mit dem Zusatzreaktor (29) in Verbindung steht, um die Melamin-Mutterlauge aus dem Melamin-Mutterlaugentank (16) in den Zusatzreaktor (29) im Guanidin-Carbonat-Verfahren einzuspeisen.

## Revendications

1. Procédé pour augmenter le rendement du carbonate de guanidine dans un procédé de mélamine (10) et procédé de traitement du carbonate de guanidine (20) combinés, par
ajout du cyanamide et/ou du dicyandiamide à
a) une liqueur mère de mélamine (MML) obtenue dans le procédé de mélamine (10), et/ou
b) une liqueur mère de carbonate de guanidine (GCML) obtenue dans le procédé de traitement de carbonate de guanidine (20), et/ou
c) un mélange d'une liqueur mère de mélamine (MML) et d'une liqueur mère de carbonate de guanidine (GCML).

2. Procédé selon la revendication 1, **caractérisé en ce que**, en plus du cyanamide et/ou du dicyandiamide, du carbonate d'ammonium ou en variante de l'ammoniac/dioxyde de carbone est ajouté à la liqueur mère de mélamine (MML), la liqueur mère de carbonate de guanidine (GCML) ou leur mélange, en particulier à la liqueur mère de carbonate de guanidine (GCML).

3. Procédé selon la revendication 1 ou 2, **caractérisé en ce que** 2,5 à 50 g/l de cyanamide et/ou 2,5 à 50 g/l de dicyandiamide et 2,5 à 100 g/l de carbonate d'ammonium sont ajoutés.

4. Procédé selon l'une des revendications précédentes, **caractérisé en ce que** 2,5 à 25 g/l de cyanamide et/ou 2,5 à 25 g/l de dicyandiamide et 2,5 à 50 g/l de carbonate d'ammonium sont ajoutés à la liqueur mère de mélamine (MML).

5. Procédé selon l'une des revendications 1 à 3, **caractérisé en ce que** 5 à 50 g/l de cyanamide et/ou 5 à 50 g/l de dicyandiamide et 5 à 100 g/l de carbonate d'ammonium sont ajoutés à la liqueur mère de carbonate de guanidine (GCML).

6. Procédé selon l'une des revendications précédentes, **caractérisé en ce que** l'ajout de cyanamide et de dicyandiamide et éventuellement de carbonate d'ammonium est réalisé à une température comprise entre 100 et 150°C, de préférence entre 110 et 140°C, pendant 15 à 360 min, de préférence entre 30 et 120 min.

7. Procédé selon l'une des revendications précédentes, **caractérisé en ce que** la liqueur mère de mélamine (MML) est obtenue dans un procédé de mélamine basse pression comprenant les étapes consistant à :
- introduire de l'urée et de l'ammoniac dans au moins un décomposeur pour obtenir un mélange décomposé contenant de l'acide isocyanique ;
- introduire le mélange décomposé contenant de l'acide isocycanique dans au moins un réacteur catalytique à lit fixe dans lequel un mélange réactionnel contenant de la mélamine est formé ;
- introduire le mélange réactionnel contenant de la mélamine dans au moins un désactivateur ;
- retirer la suspension de mélamine du désactivateur, et
- fournir la liqueur mère de mélamine (MML).

8. Procédé selon l'une des revendications précédentes, **caractérisé en ce que** la liqueur mère de mélamine (MML) comprend de l'urée, de la mélamine, de l'ammoniac et du carbonate de guanidine, en particulier ladite liqueur comprend 10 à 150 g/l, de préférence 30 à 70 g/l d'urée, 2 à 10 g/l, de préférence 4 à 6 g/l de mélamine, 50 à 300 g/l, de préférence 100 à 200 g/l, plus préférablement 140 à 150 g/l d'ammoniaque et 5 à 60 g/l, de préférence 15 à 35 g/l de carbonate de guanidine.

9. Procédé selon l'une des revendications précédentes, **caractérisé en ce qu'**une liqueur mère de carbonate de guanidine (GCML) est obtenue dans le procédé de traitement de carbonate de guanidine comprenant les étapes consistant à :
- évaporer, dans une étape d'évaporation, la liqueur mère de mélamine (MML) provenant du procédé de mélamine et la liqueur mère de carbonate de guanidine (GCML) provenant du procédé de traitement de carbonate de guanidine, dans lequel la mélamine précipite ;
- éliminer la mélamine précipitée, de préférence par filtration ;
- ajouter de l'ammoniac à la liqueur mère pour précipiter le carbonate de guanidine et éliminer le carbonate de guanidine précipité, de préférence par centrifugation, et
- fournir la liqueur mère de carbonate de guanidine (GCML), qui est réintroduite dans l'étape d'évaporation.

10. Procédé selon la revendication 9, **caractérisé en ce qu'**après l'ajout du cyanamide et du dicyandiamide et éventuellement du carbonate d'ammonium à la liqueur mère de carbonate de guanidine (GCML), la liqueur mère de carbonate de guanidine (GCML) contenant du cyanamide et du dicyandiamide et éventuellement du carbonate d'ammonium est mélangé avec une liqueur mère de mélamine fraîche (MML) et le mélange est réintroduit dans l'étape d'évaporation.

11. Procédé selon la revendication 9, **caractérisé en ce que** la liqueur mère de carbonate de guanidine (GCML) est mélangée avec une liqueur mère de mélamine fraîche (MML), ensuite du cyanamide et du dicyandiamide et éventuellement du carbonate d'ammonium sont ajoutés et le mélange est réintroduit dans l'étape d'évaporation.

12. Procédé selon la revendication 9, **caractérisé en ce que** du cyanamide et du dicyandiamide et éventuellement du carbonate d'ammonium sont ajoutés à la liqueur mère de mélamine (MML), ensuite une liqueur mère de carbonate de guanidine (GCML) est ajoutée et le mélange est réintroduit dans l'étape d'évaporation.

13. Procédé selon l'une des revendications 9 à 12, **caractérisé en ce que** 15 à 30% en poids, de préférence 20 à 25% en poids d'ammoniac gazeux ou liquide sont ajoutés à la liqueur mère pour la précipitation du carbonate de guanidine.

14. Procédé selon l'une des revendications précédentes, **caractérisé en ce que** la liqueur mère de carbonate de guanidine (GCML) comprend de l'urée, de la mélamine, de l'ammoniac et du carbonate de guanidine, de préférence ladite liqueur comprend 300 à 150 g/l, de préférence 70 à 100 g/l d'urée, 2 à 8 g/l, de préférence 2 à 5 g/l de mélamine, 100 à 350 g/l, de préférence 180 à 270 g/l, plus préférablement 200 à 250 g/l d'ammoniac et 10 à 60 g/l, de préférence 25 à 35 g/l de carbonate de guanidine.

15. Installation pour la mise en œuvre du procédé selon l'une des revendications précédentes comprenant
- des dispositifs pour la mise en œuvre du procédé de mélamine (10) avec au moins un réservoir de liqueur mère de mélamine (16) pour une liqueur mère de mélamine (MML) obtenue après la séparation de la mélamine d'une suspension de mélamine, et
- des dispositifs pour la mise en œuvre du procédé de carbonate de guanidine (20) avec au moins un réservoir de liqueur mère de carbonate de guanidine (28) pour une liqueur mère de carbonate de guanidine (GCML) obtenue après la séparation du carbonate de guanidine d'une suspension de carbonate de guanidine ; et avec au moins un réacteur d'évaporation (21) situé en aval du réservoir de liqueur mère de carbonate de guanidine (21) ;
- dans lequel entre l'au moins un réservoir de liqueur mère de carbonate de guanidine (28) et l'au moins un réacteur d'évaporation (21) un réacteur supplémentaire (29), en particulier un réacteur tubulaire ou un réacteur agité, est prévu, et
- dans lequel le réservoir de liqueur mère de mélamine (16) est également relié audit réacteur supplémentaire (29) pour amener la liqueur mère de mélamine du réservoir de liqueur mère de mélamine (16) jusque dans ledit réservoir supplémentaire (29) dans le procédé de carbonate de guanidine.
